# EUROPEAN PATENT SPECIFICATION

(11) **EP 2 823 052 B1**
(45) Date of publication and mention of the grant of the patent: **26.10.2016**
(21) Application number: 13707185.8
(22) Date of filing: 05.03.2013
(51) Int. Cl.: C12Q 1/32

(54) **COMPATIBLE SOLUTE ECTOINE AS WELL AS DERIVATIVES THEREOF FOR ENZYME STABILIZATION**
KOMPATIBLES GELÖSTES ECTOIN SOWIE DERIVATE DAVON ZUR ENZYMSTABILISIERUNG
ECTOÏNE EN SOLUTÉ COMPATIBLE AINSI QUE SES DÉRIVÉS POUR LA STABILISATION D'ENZYMES

(30) Priority: 06.03.2012 EP 12158286
(43) Date of publication of application: 14.01.2015
(73) Proprietor: Roche Diabetes Care GmbH, 68305 Mannheim (DE); F.Hoffmann-La Roche AG, 4070 Basel (CH)
(72) Inventor: CHEMNITIUS, Gabriele, 68199 Mannheim (DE); GAA, Otto, 67551 Worms (DE); NAGEL, Thomas, 68309 Mannheim (DE); RECHT, Karl, 68642 Bürstadt (DE)
(74) Representative: Herzog, Fiesser & Partner Patentanwälte PartG mbB
(86) International application number: PCT/EP2013/054351
(87) International publication number: WO 2013/131885

(56) References cited:
- WO-A1-2007/000596
- WO-A1-2008/155531
- WO-A2-2007/072013
- LIPPERT K ET AL: "ENZYME STABILIZATION BY ECTOINE-TYPE COMPATIBLE SOLUTES: PROTECTION AGAINST HEATING, FREEZING AND DRYING", APPLIED MICROBIOLOGY AND BIOTECHNOLOGY, SPRINGER VERLAG, BERLIN, DE, vol. 37, no. 1, 1 April 1992 (1992-04-01), pages 61-65, XP000608489, ISSN: 0175-7598, DOI: 10.1007/BF00174204

## Description

The present invention is concerned with means and methods for maintaining and preserving enzymatic activity. In particular, the invention relates to a dry composition comprising a dehydrogenase, a redox cofactor, an agent capable of eliciting at least one optical change in an optical property of an indicator reagent in the presence of redox equivalents, an indicator reagent, and at least one compatible solute being ectoine or a derivative thereof. The invention further contemplates a diagnostic test element for the determination of an analyte from a body fluid sample and a method for the manufacture of such a test element. Further envisaged by the present invention is the use of at least one compatible solute as mentioned above for reducing a decrease of the enzymatic activity of at least one enzyme in a composition under dry conditions. Furthermore, contemplated is a method for determining the presence or amount analyte in a body fluid sample based on the test element according to the invention.

Diagnostic test elements are usually manufactured for use in near-patient applications. Therefore, the elements must be robust with respect to handling and storage. This applies, in particular, for the test chemistry of the test elements. (see Hönes 2008, Diabetes Technology & Therapeutics 10: S10)

However, many diagnostic test elements are based on a rather complex enzyme test chemistry present on the test element. In particular, the test element comprises a carrier and a detection layer wherein the detection layer usually contains enzymes. It is decisive for the proper function of the test elements that these enzymes remain biologically active during storage and upon treatment. Since calibration for an individual measurement is usually not possible, the test elements are normally calibrated batch-wise. The calibration information for a batch of test elements is stored and used for each element of the batch regardless of individual differences in treatment and storage.

However, pretreatments of the test elements and storage conditions can severely affect the activity of the enzymes. For example, heat treatment, either during the manufacture or the storage of the test elements, can denature the enzymes such that the overall enzymatic activity present on a test element is significantly reduced which, in turn, will result in wrong test results when such a test element is used. Similarly, many enzymes are sensitive with respect to oxidation processes which also results in denaturation and irreversible enzyme inactivation. However, many enzymes on test elements are present, at least during the time of storage, in a solvent-free environment which may promote even such oxidation processes. Moreover, the detection layer may comprise additional components which even more facilitate oxidation processes such as redox cofactors and other redox relevant components.

The problem of preserving enzymatic activities under such unfavorable conditions, however, does not only apply for test elements. Rather, more generally, many enzyme preparations are provided and stored in essentially solvent-free form, such as freeze-dried preparations.

Various so-called compatible solutes, i.e. low molecular compounds of different chemical classes, such as sugars, polyols, free amino acids, amino acid derivatives, amines and sulphur analoga, sulfateesters, short peptides and cyclic 2,3-diphosphoglycerate, have been investigated for their preservative properties for proteins in solution and under dry conditions (see Arakawa 1985, Biophys J 47: 411; Lippert 1992, Appl Microbiol Biotechnol 37: 61; Göller 1999, J Mol Catal B Enzymatic 7:37; Lentzen 2006, Appl Microbiol Biotechnol 72: 623; WO2007/002657, US2010/0255120).

Stabilisation of glucose oxidase by ectoine and hydroxyectoine in biosensors for the electrochemical determination of glucose in solution has been previously reported (WO2007/097653; Loose 2006, Proceedings of the 24th IASTED International Multi-conference Biomedical Engineering, 167-173, Innsbruck, AT). However, glucose oxidase is known to be a rather stable enzyme with respect to oxidative stress and heat. On the other hand, hydroxyectoine was reported to be incapable of preventing protein aggregation in solution for lactate dehydrogenase (Andersson 2000, Biotechnol Appl Biochem 32: 145-153). Dehydrogenases in general and, in particular, glucose dehydrogenases are rather sensitive enzymes for oxidative stress and heat treatment. However, they are important diagnostic tools.

Ectoine has also been reported as agent in Cholesterol biosensors comprising cholesterol dehydrogenase aiming for electrochemical detection of the enzymatic activity (WO2007/132226 or WO2006/067424).

However, there is a need for preservation of the enzymatic activity of storage and temperature sensitive enzymes used for diagnostic applications and, in particular, for the class of dehydrogenases, such as glucose dehydrogenase.

The technical problem underlying the present invention could be regarded as the provision of means and methods for complying with the aforementioned needs. The problem is solved by the embodiments characterized in the claims and herein below.

Thus, the present invention relates to a dry composition as disclosed in the appended claims 1 to 5.

The term "dry" as used herein means that the composition is essentially free of a solvent or a mixture of solvents. Essentially free as used herein means that at least 85%, at least 90%, at least 92%, at least 95%, or at least 98% of the solvent or solvent mixture which was originally present in a solution of the composition has been removed from the composition. Accordingly, it is, preferably, envisaged that the solvent or solvent mixture is present in the dry composition of the invention in an amount of up to 15%, and preferably, up to 10%, up to 8%, up to 5%, up to 2%. The aforementioned percentage values and the other percentage values referred herein used in order to define amounts refer to percent by weight (w/w).

Such a composition of the invention is, preferably, a solid composition under normal conditions, i.e. under room temperature and normal pressure.

A solvent in accordance with the present invention is a solvent which allows for dissolving the components of the composition of the present invention under conditions which do not irreversibly impair the function of the components of the composition and, in particular, the enzymatic activity of the dehydrogenase. Moreover, it is envisaged that the solvent dissolves the components, preferably, under standard pressure, preferably 1 bar +/- 10% within a temperature range of 5 to 40°C and, more preferably, room temperature, preferably 20°C +/-10°C. Suitable solvents according to the present invention include, preferably, water, water-based buffers, such as phosphate buffered saline or Tris buffers, alcohols, such as hexanol, 2-methoxy-propanol, 2-methyl-2-butanol, citrate buffer, glycerine phosphate, or Good's buffer (preferably in addition to Tris buffer). It will be understood that in accordance with the present invention, a solvent to be used can be a mixture of two or more of the aforementioned solvents. Preferred solvent mixtures envisaged in this context are mixtures of water or water-based buffers with alcohols and in particular, the solvent mixtures referred to in the accompanying Examples, below.

A dry composition according to the present invention can be provided, preferably, by dissolving the components of the composition of the present invention first in a solvent or mixture of solvents and subsequently removing the said solvent or mixture of solvents by a suitable treatment such that the remaining composition is essentially free of the said solvent or solvent mixture. Suitable treatments to be preferably envisaged by the present invention include heat treatment, evaporation techniques, freeze drying. Preferably, the envisaged treatment is heat treatment and, in particular, heat treatment under the following conditions: heat treatment at about 60°C or more for approximately 20 to 45 minutes or at about 95°C for approximately 1 to 2 minutes with heat circulation; thickness of the composition of 20 to 200 micrometers or less; at a pressure of 1 bar or 0.1 bar. Moreover, it will be understood that in order to keep the composition under dry conditions, storage is, preferably, carried out in the presence of a drying agent. Suitable drying agents, preferably, encompass silica gel, zeolites, calcium carbonate or magnesium sulfate.

The term "dehydrogenase" as used herein refers to polypeptides which are capable of catalyzing the oxidation of a substrate by transferring hydrides (H⁻) as redox equivalents to an acceptor molecule, preferably, to a redox cofactor as referred to herein elsewhere. Dehydrogenases envisaged by the present invention are, preferably, those which depend on a redox cofactor (or sometimes referred to as co-enzyme) such as pyrrolo quinoline quinone (PQQ), nicotinamide-adenine-dinucleotide (NAD) or a derivative thereof, or a flavine cofactor, such as flavin-adenine-dinucleotide (FAD) or flavine mononucleotide (FMN). Preferred dehydrogenases are, in particular, lactate dehydrogenase (EC number 1.1.1.27 or 1.1.1.28), glucose dehydrogenases (see below), alcohol dehydrogenase (EC number EC number 1.1.1.1 or 1.1.1.2), L-amino acid dehydrogenase (EC number 1.4.1.5), glycerin dehydrogenase (EC number 1.1.1.6), malate dehydrogenase (EC number 1.1.1.37), 3-hydroxybutyrat dehydrogenase (EC number 1.1.1.30), or sorbitol dehydrogenase (EC number 1.1.1.14).

The dehydrogenase of the invention is a glucose dehydrogenase. Most preferably, said glucose dehydrogenase is selected from the group consisting of: glucose dehydrogenase (EC number 1.1.1.47), quinoprotein glucose dehydrogenase (EC number 1.1.5.2), in particular, Pyrrolo quinoline quinone (PQQ)-dependent glucose dehydrogenase (EC number 1.1.5.2), glucose-6-phospate dehydrogenase (EC number 1.1.1.49), nicotinamide adenine dinucleotide (NAD)-dependent glucose dehydrogenase (EC number 1.1.1.119) and flavin adenine dinucleotide (FAD)-dependent glucose dehydrogenase (EC number 1.1.99.10) or enzymatically active mutants thereof. Preferred in accordance with the present invention is a glucose dehydrogenase (E.C. 1.1.1.47) mutant disclosed in WO2011/020856 having a mutation at least at amino acid position 96, 170 and/or 252, herewith incorporated by reference. Preferred mutations envisaged at theses amino acid positions are substitutions of Glu96Gly, Glu170Arg or Lys and/or Lys252Leu.

The structure and properties of preferred members of the said enzyme families is well known in the art (see Olsthoorn 1998, Biochemistry 37: 13854-13861; Pauly 1976, Hoppe Seylers Z Physiol Chem 356: 1613-1623; Tsujimura 2006, Biosci Biotechnol Biochem 70: 654-659). Enzymatically active mutants of the aforementioned enzymes can be obtained by substituting, adding or deleting one or more amino acids from the amino acid sequences reported for the aforementioned wild type enzymes in the prior art as recited before. Preferred mutants are the mutants of the PQQ-dependent glucose dehydrogenase having an improved substrate specificity compared to their wild type counterparts as disclosed in US 7,132,270 or US 7,547,535. Both documents are herewith incorporated by reference with respect to the mutants. Further mutants are those disclosed in Baik et al (Baik 2005, Appl Environ Microbiol 71: 3285), Vasquez-Figuera et al. (Vasquez-Figuera 2007, Chem BioChem 8: 2295), und WO 2005/045016.

The term "redox cofactor" as used herein refers to a molecule which can serve as an acceptor for enzymatically transferred redox equivalents and, in particular, hydride (H-). Preferably, the redox cofactor is PQQ, NAD or FAD. It will be understood that the redox cofactor to be included in the composition of the present invention depends on the properties of the dehydrogenase to be envisaged. For example, PQQ is combined in a composition according to the present invention with a PQQ dependent glucose dehydrogenase, NAD is combined in a composition according to the present invention with a NAD dependent glucose dehydrogenase, and FAD is combined in a composition according to the present invention with a FAD dependent glucose dehydrogenase. A redox cofactor according to the present invention may also preferably be a derivative of PQQ, NAD or FAD. Preferred derivatives of NAD are those disclosed in WO 2007/012494 which is herewith incorporated by reference with respect to the disclosed NAD/NADH and/or NADP/NADPH derivatives. More preferably, the redox cofactor in accordance with the present invention is carba-NAD as disclosed in WO 2007/012494.

Ectoine (CAS number: 96702-03-3) is a well-known organic compound which naturally occurs in bacteria having the formula C₆H₁₀N₂O₂. It is also referred to as (S)-2-methyl-3,4,5,6-tetrahydropyrimidine-4-carboxylic acid. Ectoine is obtainable from various bacteria, in particular, of the genera Halomonadaceae or Firmicutes, by techniques well known in the art (see WO1994/15923).

A "derivative" of ectoine as referred to in accordance with the present invention is a structurally related organic molecule capable of preventing a reduction of enzymatic activity of at least one enzyme as referred to herein in solution as well as in the dry state. Preferably, the said reduction of the enzymatic activity is prevented in a similar or the same manner and/or to a similar or the same extent as found for ectoine. More preferably, the derivative of ectoine, if present in a composition according to the invention, shall be capable of preventing the reduction of enzymatic activity which occurs during manufacture and/or storage, in particular storage at room temperature or even higher temperatures such as 45°C or 50°C or even temperatures up to 60°C, to a statistically significant extent. Preferably, the derivative, if present in the composition, shall be capable of maintaining at least 60%, at least 70%, at least 80% or at least 90% of the enzymatic activity of at least one enzyme in the composition compared to the enzymatic activity found in a control composition without the said ectoine derivative. The prevention of the reduction of the enzymatic activity under dry conditions can be determined as described elsewhere herein, e.g., in the accompanying Examples. Preferably, said derivative of ectoine is selected from the group consisting of: hydroxyectoine, homoectoine, a hydroxyectoine ester, a hydroxyectoine ether, a sulfonyl derivative of ectoine, an esterified sulfonyl derivative of ectoine and an amide of a sulfonyl derivative of ectoine. As for ectoine, hydroxyectoine is obtainable from various bacteria, in particular, of the genera Halomonadaceae or Firmicutes, by techniques well known in the art (see WO1994/15923). The other aforementioned ectoine derivatives can be obtained, e.g., by chemical derivatizing hydroxyectoine in vitro.

At least one compatible solute as referred to herein means that two or more compatible solutes may be used together in the composition of the present invention. Preferably, ectoine and one or more derivatives thereof can be applied in the composition of the invention. Hydroxyectoine is a preferred derivative in this context. Moreover, a combination of derivatives of ectoine, such as hydroxyectoine and homoectoine, may also be applied.

The term "redox equivalents" as used herein refers to hydrides (H⁻) which are transferred from a substrate of the dehydrogenase to the redox cofactor or electrons transferred to the indicator reagent from the redox cofactor.

The term "agent capable of eliciting a change in at least one optical property of an indicator reagent in the presence of redox equivalents" refers to a molecule which in the presence of the redox equivalents is capable of inducing a change in at least one optical property in an indicator reagent. It is to be understood in accordance with the present invention that the agent may also elicit a change in more than one optical properties of the indicator reagent which may than subsequently detected. A optical property as referred to herein refers to a property of the indicator reagent which can be optically detected such as light absorption or emission, remission, refraction or polarization and properties associated therewith. It will be understood that such a change of at least one optical property as used herein encompasses the detection of the presence of a property which was not detectable before, the detection of the absence of a property which has been detected before and the detection of quantitative changes of a property, i.e. the detection of the change of the signal strength which correlates to the extent of the change of the at least optical property. Preferred optical properties envisaged by the present invention are color, fluorescence, luminescence, or refractometry. The optical properties which are to be changed by the agent envisaged according to the present invention depend on the type of indicator reagent. Dependent on the desired optical property to be detected and the agent to be used in the composition, the skilled person is in a position to select without further ado a suitable indicator reagent, in particular among those referred to herein elsewhere.

An agent as referred to above is, preferably, capable of transferring directly or indirectly, i.e. via a further mediator, redox equivalents from the redox cofactor to the indicator reagent. As a consequence of the said transfer of the redox equivalents, the indicator reagent will be modified such that a change in at least one optical property occurs. For example, a color-less or non-fluorescing indicator reagent in an oxidized state may be converted into a colored or fluorescent indicator reagent by the transfer of redox equivalents mediated by the agent in a reduced state. The transfer of the redox equivalents may be direct in that the redox equivalents are transferred by the agent to the indicator reagent or may be indirect. In the latter case, the redoxequivalents are transferred from the agent to an intermediate mediator which subsequently transfers the redox equivalents to the indicator reagent. It will be understood that, preferably, more than one mediator can be used. For example, the agent may transfer the redox equivalents to a first mediator which subsequently transfers the redox equivalents to a second mediator and said second mediator than transfers the redox equivalents to the indicator reagent. It will be understood that in such a mediator cascade more than two mediators could be used. An advantage of using one or more mediators for the transfer of the redox equivalents to the indicator reagent is that the timing of the optical detection can be improved.

Mediators which can be applied in the context of the present invention are well known in the art and include, e.g., potassium ferricyanide, quinone derivatives, Nile blue (CAS no.: 3625-57-8), Meldola's blue (CAS no.: 7057-57-0), osmium complexes as disclosed in EP 1 457 572 B1, herewith incorporated by reference, or transition metal complexes such as ruthenium hexamine chloride.

An agent according to the invention which is, preferably, envisaged is a phenazine. More preferably, said phenazine is phenazinethosulfate, phenazinmethosulfate, 1-(3-carboxypropoxy)-5-ethylphenaziniumtrifluoromethansulfonate or 1-methoxyphenazinmethosulfate. Such phenazines can be applied for eliciting a change in at least one optical property of an indicator reagent. Details for the detection and on how such phenazines are to be applied can be found in EP 0 654 079 A1 which is herewith incorporated by reference.

Also, an agent envisaged in this context is, preferably, a chinone. More preferably, the said chinone is phenanthrenchinone, phenanthrolinchinone or benzo[h]-chinolinchinone.

Another agent envisaged in this context is a nitrosoaniline. More preferably, said nitrosoaniline is [(4-nitrosophenyl)imino]dimethanol-hydrochloride.

Also preferably envisaged by the present invention is an agent capable of eliciting a change in at least one optical property of an indicator reagent in the presence of redox equivalents, said agent being an enzyme capable of catalyzing the transfer of redox equivalents from the redox cofactor to the indicator reagent. More preferably, the enzyme envisaged in this context according to the present invention is a diaphorase (EC number 1.6.99.2), preferably, a lipoamide deydrogenase or a NADH dehydrogenase or an enzymatically active mutant thereof. Preferred diaphorases are those from pig heart, Clostridium kluyverii or Bacillus stearothermophilus. The structure of the said enzymes is well known in the art and described, e.g., in DE 2 061 984. Enzymatic active mutants can be provided as described elsewhere herein. Particular preferred diaphorases envisaged in accordance with the present invention are those having improved thermostability and catalytic properties as disclosed in US2007/196899 which is herewith incorporated by reference with respect to the corresponding disclosure content.

Preferably, the compatible solute reduces a decrease of the enzymatic activity of at least one enzyme in the composition under dry conditions. More preferably, said at least one enzyme is the dehydrogenase. More preferably, the at least one enzyme can be, however, also both, the dehydrogenase as well as an enzyme, preferably a diaphorase as specified above, which is applied in the composition of the present invention as an a agent capable of eliciting a change in at least one optical property of an indicator reagent in the presence of redox equivalents. More preferably, the decrease of the enzymatic activity is prevented or at least significantly reduced in the composition of the invention during manufacture and/or storage at room temperature or even higher temperatures as referred to above by the at least one compatible solute in comparison to a control composition without said compatible solute such that at least 60%, at least 70%, at least 80% or at least 90% of the enzymatic activity of one or both enzymes is maintained.

The term "indicator reagent" as used herein refers to a molecule or molecular entity which as a consequence of the transfer of redox equivalents will be modified such that a change in at least one optical property occurs.

Preferred indicator reagents to be applied in the composition of the invention encompass heteropoly acids, preferably, 2,18-phosphoromolybdenic acid, chinones, preferably resazurine, dichlorophenolindophenole, and/or tetrazolinum salts, preferably, the commercially available WST-3, WST-4 and WST-5 salts (Dojindo, Inc. US). These indicator reagents are reduced upon transfer of the redox equivalents and this reduction is accompanied by a change in at least one optical property and, in particular, the color.

Further preferred indicator reagents envisaged in accordance with the present invention are reagents, such as fluorophores, the fluorescence of which is changed upon transfer of redox equivalents. Suitable fluorophores include, among others, the flavine nucleotides and nicotin-adenine-dinucleotides referred to herein also in the context of the redox cofactors. If a redox cofactor such as carba-NAD or NAD is applied in the composition of the invention as an indicator reagent, the components (b), (c) and (d) of the composition of the invention may all be represented by the same molecule, i.e. the carba-NAD or NAD. Accordingly, the components (b), (c) and (d) may be represented in the composition of the invention by the same chemical entity. Moreover, a modified nitrosoaniline as disclosed in EP 0 620 283 B1 or EP 0 831 327 B1, the respective disclosure content of which is herewith incorporated by reference, can be used, preferably, as component (c) and component (d). Thus, components (c) and (d) may be represented in the composition of the invention by the same chemical entity.

The procedures for removing solvent from a composition referred to herein above and, in particular, the heat treatment, are known to affect the enzymatic activity, in particular, the enzymatic activity of sensitive enzymes such as dehydrogenases. Moreover, under dry conditions, enzymes such as dehydrogenases become more sensitive for oxidation processes and accompanying enzyme denaturation (Andersson 2000, Biotechnol. Appl. Biochem 32: 145-153). Accordingly, the manufacture of complex, dry compositions for enzymatic detection assays as well as the storage thereof is often accompanied by increasing inactivation of the enzymes by denaturation, aggregation or other processes. Upon reconstitution of the enzymes in a solvent-containing surrounding, a reduced enzymatic activity is often observed. Advantageously, it has been found in the studies underlying the present invention that the reduction of the said enzymatic activity which occurs during manufacture and/or storage of the complex dry compositions of the present invention can be significantly prevented by the addition of the at least one compatible solute as specified elsewhere herein in detail. The finding is surprising since ectoine and derivatives thereof have been reported to be insufficient to prevent aggregation of, e.g., lactate dehydrogenase which also results in a reduction of enzymatic activity (Andersson 2000, loc cit.). Moreover, it is also surprising that the preservative effect occurs even under the redox sensitive conditions present in the rather complex, solvent-free composition of the present invention. Interestingly, and also surprisingly, a preservative effect of ectoine was not observed in solution for the investigated dehydrogenases. In particular, the enzymatic activity present in a dry composition having the components of the composition of the invention except for the at least one compatible solute has been found in the studies underlying the present invention to decrease in the dry state and during storage under dry conditions and temperatures above 4°C. In particular, it has been found that only about 50% of the enzymatic activity of, e.g., a glucose dehydrogenase is present after 3 weeks storage at 45°C and only about 55% of the enzymatic activity of, e.g., a diaphorase is maintained at said conditions. However, significantly higher enzymatic activities could be maintained in the studies underlying the present invention when a at least one compatible solute being ectoine or a derivative thereof was present in the composition. A further advantage of the composition of the present invention is that the compatible solute applied in the composition does not interfere with the optical detection system. In particular, it does not interfere with the optical signals generated and also does not impair the stability or function of the indicator reagent, the redox cofactor or the agent capable of eliciting the at least one optical change. Moreover, the enzymatic conversion and conversion rates are, preferably, not impaired by the compatible solute.

In a preferred embodiment of the composition of the present invention, the compatible solute is present in amounts of at least 3 (w/w)%, at least 4 (w/w)%, at least 5 (w/w)%, at least 6 (w/w)%, at least 7 (w/w)% or at least 8 (w/w)%.

In a preferred embodiment of the composition of the present invention, said composition further comprises at least one stabilizer, detergent, swelling agent, film-forming agent, and/or solid particle. Suitable stabilizers, detergents, swelling agents, film forming agents, oxidizing agents, and/or solid particles to be used in the composition of the invention are known to the skilled artisan.

Preferably, the said at least one stabilizer is polyvinylpyrrolidone, and, more specifically, PVP K25.

Preferably, the said at least one detergent is selected from the group consisting of: Sodium-N-methyl-N-oleoyltaurat, N-octanoyl-N-methyl-glucamid, Mega 8 (N-methyl-N-octanoylglucamide), dioctylsodium sulfosuccinate (DONS), Rhodapex® (preferably CO-433 or CO-436).

Preferably, said at least one swelling agent is selected from the group consisting of: methyl vinyl ether maleic acid anhydride copolymer, xanthan gum and methyl vinyl ether maleic acid copolymer.

Preferably, said at least one film-forming agent is selected from the group consisting of: polyvinylpropionate dispersions, Polyvinyl esters, polyvinyl acetates, polyacrylic esters, polymethacrylic acid, polyvinyl amides, polyamides, polystyrene and mixed polymerizates are also suitable such as of butadiene, styrene or maleic acid ester.

Preferably, said at least one solid particle is selected from the group consisting of: silica particles, in particular, silicon dioxide, sodium silicates or aluminium silicates, kieselguhr, metal oxides, in particular, titan oxide and/or aluminium oxide, synthetic oxide materials, in particular, nanoparticles of oxide materials such as nanoparticles of silicon dioxide, aluminium oxide, or titan oxide, Kaolin, powder glass, amorphous silica, calcium sulfate, and barium sulfate.

In a particular preferred embodiment of the composition of the invention, said composition comprises and, even more preferably, essentially consists of the components listed in Table 1 in the accompanying Examples.

All definitions and explanations for the terms made herein above apply mutatis mutandis for the embodiments described as follows. Additional definitions and explanations made further below also apply for all embodiments described in this specification mutatis mutandis.

The present invention relates to a diagnostic test element for the determination of an analyte from a body fluid sample comprising the composition of the present invention and a carrier.

The term "carrier" used in the context of the present invention refers to a solid support onto which the composition of the present invention can be applied. Preferably, the composition can be immobilized on the carrier. Moreover, it is also envisaged that the composition can be spatially arranged on the carrier. The carrier must be arranged in a manner as to allow for the detection of the change of the at least one optical property of the indicator reagent, i.e. it preferably does not comprise components or a spatial arrangement which would interfere with the detection of the at least one optical property. Suitable carriers may comprise vials containing the composition of the present invention, e.g., vials arranged in a well-plate format. Other assays may apply optical waveguides or semiconductor plates. Preferred carriers, however, are those used for test strips. Said test stripes usually comprise one or more layers forming a solid carrier.

The term "body fluid sample" as used herein refers to all body fluids known or suspected to comprise the analyte to be determined. Preferred body fluids known to comprise a plurality of diagnostically relevant analytes are blood including whole blood, plasma and serum, urine, saliva, liquor, synovial liquid, and sudor. More preferably, the body fluid sample in accordance with the present invention is a whole blood sample.

The term "analyte" as used herein refers to a biological molecule present in the body fluid sample the presence, absence or amount of which shall be determined in accordance with the present invention. Since the determination described herein is based on the enzymatic activity of a dehydrogenase, it will be understood that the said analyte is a substrate of the dehydrogenase comprised by the composition. Preferred analytes envisaged to be determined in accordance with the present invention, e.g., by the aforementioned diagnostic test element are glucose, maltose, mannose, galactose, glutamate, glucose-6-phosphate, ethanol or lactose and, more preferably, glucose.

Preferably, the diagnostic test element of the invention comprises a test field containing said composition, wherein the test field has a sample application side onto which the body fluid sample is applied and a detection side which allows for detection of a change in an optical property when the analyte reacts with the composition. The sample is to be applied to the sample application side and it is, preferably, envisaged that cells, such as erythrocytes present in blood samples, do not reach the detection side.

Details on such a test element and the manufacture thereof can be found in EP 0 821 234 B1 which is herewith incorporated by reference. Further test elements envisaged in accordance with the present invention are those disclosed in EP 1 035 919 B1 or EP 1 035 920 B1, the respective disclosure content of which is herewith incorporated by reference..

Specifically, the test field of the diagnostic test element of the invention comprises, preferably, a transparent foil onto which a first and a second film layer are applied resting on top of one another in this order. It is important that the first layer located on the transparent foil scatters light considerably less than the overlying second layer. The non-coated side of the transparent foil is referred to as the detection side and the side of the second layer which is opposite to the side with which the second layer rests on the first layer is referred to as the sample application side.

The film layers of the diagnostic test element according to the invention are produced from dispersions or emulsions of polymeric film formers. Dispersion film formers contain microscopic polymer particles which are insoluble in the carrier liquid (usually water) and are finely dispersed in the carrier liquid. If the liquid is removed by evaporation during film formation then the particles come closer and finely touch one another. The large forces which occur in this process and the gain in surface energy which accompanies the film formation results in the particles growing into a substantially closed film layer. Alternatively it is also possible to use an emulsion of the film former in which this is dissolved in a solvent. The dissolved polymer is emulsified in a carrier liquid which is immiscible with the solvent. Polyvinyl esters, polyvinyl acetates, polyacrylic esters, polymethacrylic acid, polyvinyl amides, polyamides and polystyrene are particularly suitable as polymers for such film formers. In addition to homopolymers mixed polymerizates are also suitable such as of butadiene, styrene or maleic acid ester.

The two so-called film layers are located on a transparent foil in the test field of the diagnostic test carrier according to the invention. For this those plastic foils come into consideration which are impermeable to liquid. Polycarbonate foil has proven to be particularly suitable.

The two film layers can be produced from coating compounds which contain the same polymeric film formers or they can be produced from coating compounds which contain different polymeric film formers.

Whereas the first layer contains a swelling agent and optionally a weakly light scattering filler, the second layer requires a swelling agent and in any case at least one pigment that scatters light strongly. In addition the second layer can also contain non-porous fillers as well as porous fillers.
By adding a swelling agent that swells well (i.e. a substance which increases its volume when it takes up water) one does not only obtain layers which can be penetrated relatively rapidly by sample liquid but have good cell, e.g., erythrocyte and additionally also blood pigment, separation properties despite this opening effect of the swelling agent. The swelling properties should be so good that for a test in which the change of the at least one optical property is mainly dependent on the penetration of the sample liquid through the layer, the change of the optical property is measurable after a maximum of one minute. Especially suitable swelling agents have proven to be methyl vinyl ether maleic acid anhydride copolymer, xanthan gum and methyl vinyl ether maleic acid copolymer.

The amount of the strongly light-scattering pigment in the second layer is at least 25% by weight relative to the dry ready-to-use double layer of the test field. Since the weakly light-scattering fillers and the strongly light-scattering pigments are essential for the optical properties of the film layers, the first and the second film layer have different fillers and pigments.

The first film layer should either contain no fillers or those fillers whose refractive index is near to the refractive index of water. Silicone dioxide, silicates and aluminium silicates have proven to be particularly suitable for this. A sodium aluminium silicate with the commercial name Transpafill.RTM®. is particularly preferred. It has an average composition of 66% by weight SiO2, 26% by weight Al2 03, 7% by weight Na2 O and 1% by weight SO3. The average granulate size of particularly preferred primary particles is about 0.06 µm.

According to the invention the second layer should scatter light very strongly. Ideally the refractive index of the pigments in the second film layer should be at least 2.5. Hence titanium dioxide is preferably used. Particles with an average diameter of about 0.2 to 0.8 µm have proven to be particularly advantageous. Easily processable titanium dioxide types in the anatase modification are quite especially preferred.

It is possible that the composition of the invention is comprised in one film layer, preferably, the first film layer. However, it is also possible that the composition of the present invention is present in both film layers.

In order to optimize the test field in the diagnostic test carrier according to the invention, it has proven to be particularly advantageous when both film layers do contain a non-haemolyzing wetting agent. Neutral i.e. non-charged wetting agents are particularly preferred for this. N-octanoyl-N-methyl glucamide is most particularly preferred.

In order to produce a test field of a diagnostic test element according to the invention the respective film layers are each produced successively from a homogeneous dispersion of the said components. For this the transparent foil is used as a base to form the coating compound for the first film layer. After the coating compound for the first film layer has been applied with a particular layer thickness, the layer is dried. Afterwards the coating compound for the second layer is applied to this layer also with a thin layer thickness and dried. After the drying the thickness of the first and second film layer should be together no more than 0.20 mm, preferably no more than 0.12 mm, particularly preferably no more than 0.08 mm.

The test field produced in this manner can be mounted on a supporting layer for better handling, those materials coming into consideration for such a layer which do not take up the liquid to be examined. These are so-called non-absorptive materials, plastic foils for example made of polystyrene, polyvinyl chloride, polyester, polycarbonate or polyamide being particularly preferred. Metal foils or glass are suitable as further supporting materials.

In a preferred embodiment of the test element according to the invention the detection side of the test field which is to be observed and measured for a change in at least one optical property of the indicator reagent should be visible through the supporting layer in order to determine the analyte to be detected in the body sample. This can be achieved by a transparent supporting layer. However, it is also possible that the supporting layer has a perforation which is covered by the detection side of the test field. The detection side is then visible through the perforation. Preferably, in the diagnostic test element according to the invention there is a hole in the supporting layer below the detection side of the test field through which the detection side of the test field can be observed. The hole has a somewhat smaller diameter than the smallest linear dimension of the test field so that the test field outside the hole rests on the supporting layer and can be attached there.

The present invention further relates to a method for the manufacture of a diagnostic test element comprising the step of generating a composition according to the present invention on a solid carrier.

In a preferred embodiment of the said method of the invention, said generating comprises the steps of:
(i) applying a composition comprising the components (a) to (e) of the composition of the invention and a solvent (i.e. a composition comprising the components in a dissolved state rather than being dry) to a test field on the carrier; and
(ii) removing the said solvent from the composition;
or
(i) applying a composition comprising components (a), (b), (d) and (e) of the composition of the invention and a solvent (i.e. a composition comprising the components in a dissolved state rather than being dry) to a test field on the carrier in a first layer;
(ii) removing the said solvent from the composition of the first layer;
(iii) applying a composition comprising components (c) to (e) of the composition of the invention and a solvent (i.e. a composition comprising the components in a dissolved state rather than being dry) in a second layer on the first layer; and
(iv) removing the said solvent from the composition of the second layer;
or
(i) applying a composition comprising components (a), (b), (d) and (e) of the composition of the invention and a solvent (i.e. a composition comprising the components in a dissolved state rather than being dry) to a test field on the carrier in a first layer;
(ii) removing the said solvent from the composition of the first layer;
(iii) applying a composition comprising components (b) to (e) of the composition of the invention and a solvent (i.e. a composition comprising the components in a dissolved state rather than being dry) in a second layer on the first layer; and
(iv) removing the said solvent from the composition of the second layer;
or
(i) applying a composition comprising components (a), (d) and (e) of the composition of the invention and a solvent (i.e. a composition comprising the components in a dissolved state rather than being dry) to a test field on the carrier in a first layer;
(ii) removing the said solvent from the composition of the first layer;
(iii) applying a composition comprising components (b) to (e) of the composition of the invention and a solvent (i.e. a composition comprising the components in a dissolved state rather than being dry) in a second layer on the first layer; and
(iv) removing the said solvent from the composition of the second layer;
or
(i) applying a composition comprising components (c) to (e) of the composition of the invention and a solvent (i.e. a composition comprising the components in a dissolved state rather than being dry) to a test field on the carrier in a first layer;
(ii) removing the said solvent from the composition of the first layer;
(iii) applying a composition comprising components (a), (b), (d) and (e) of the composition of the invention and a solvent (i.e. a composition comprising the components in a dissolved state rather than being dry) in a second layer on the first layer; and
(iv) removing the said solvent from the composition of the second layer.
or
(i) applying a composition comprising components (b) to (e) of the composition of the invention and a solvent (i.e. a composition comprising the components in a dissolved state rather than being dry) to a test field on the carrier in a first layer;
(ii) removing the said solvent from the composition of the first layer;
(iii) applying a composition comprising components (a), (b), (d) and (e) of the composition of the invention and a solvent (i.e. a composition comprising the components in a dissolved state rather than being dry) in a second layer on the first layer; and
(iv) removing the said solvent from the composition of the second layer.
or
(i) applying a composition comprising components (b) to (e) of the composition of the invention and a solvent (i.e. a composition comprising the components in a dissolved state rather than being dry) to a test field on the carrier in a first layer;
(ii) removing the said solvent from the composition of the first layer;
(iii) applying a composition comprising components (a), (d) and (e) of the composition of the invention and a solvent (i.e. a composition comprising the components in a dissolved state rather than being dry) in a second layer on the first layer; and
(iv) removing the said solvent from the composition of the second layer.

Preferably, the composition is applied to a detection layer in the test field. The detection layer can be generated in particular by means of at least one wet chemical process, more particularly from one or more dispersions, preferably aqueous dispersions of the composition of the present invention. Such layer-forming processes from one or more dispersions are known in principle to a person skilled in the art, and reference can be exemplarily made in turn to, for example, the abovementioned prior art, more particularly EP 0 821 234 B1.

The solvent can be removed from the composition after application of the said composition to the test field of the test element by all techniques known for removing solvents including heat treatment, evaporation or freeze-drying. Preferably, said solvent in step (b) is substantially removed by heat treatment.

Also preferably, said compatible solute reduces the decrease of the enzymatic activity of the at least one enzyme in the composition and, in particular, during the substantial removal of the solvent in step (b) which is achieved, e.g., by heat treatment and during the maintenance of the composition under dry conditions on the test element.

The present invention also contemplates, in principle, the use of at least one compatible solute being ectoine or a derivative thereof for preventing a reduction of the enzymatic activity of at least one enzyme in a composition under dry conditions, wherein said composition comprises a dehydrogenase, a redox cofactor, an agent capable of eliciting a change in at least one optical property or an indicator reagent in the presence of redox equivalents, and an indicator reagent.

Preferably, said at least one compatible solute in the composition under dry conditions is comprised in a diagnostic test element, preferably a diagnostic test element as specified above in detail.

The present invention also relates to a method for determining the presence or amount of an analyte in a body fluid sample comprising the steps of:
(a) contacting the diagnostic test element of the invention with a body fluid suspected to comprise the analyte under conditions suitable for transforming the at least one enzyme to the reconstituted state;
(b) measuring a change in at least one optical property of the indicator reagent in the wetted composition comprising the at least one enzyme in the reconstituted state on the diagnostic test element, whereby the presence or amount of the analyte in the body fluid sample will be determined.

As set forth elsewhere herein already, depending on the substrate specificity of the dehydrogenase to be used in the composition on the diagnostic test element, different analytes can be determined by the method of the invention.

Contacting as used herein means that the body fluid sample is applied to the carrier in a manner as to allow for physical contact of the composition of the invention comprised by the carrier and the body fluid sample. In particular, contacting is carried out for a time and under conditions being sufficient for allowing the dehydrogenase to be reconstituted, i.e. wetted and dissolved, and, thus, to become biologically active. Suitable conditions depend on the diagnostic carrier and are known in the art. The body fluid sample applied to the test element, preferably, has a volume of less than 2 microliters, more particularly of less than 1 microliter.

Upon reconstitution of the said biologically active dehydrogenase, the enzyme shall bind to its substrate, i.e. the analyte comprised in the body fluid sample, and convert it into the respective product and redox equivalents. The redox equivalents generated by the dehydrogenase allow for determining the dehydrogenase activity since the redox equivalents generated by the enzymatic conversion catalyzed by the dehydrogenase are transferred by the agent capable of eliciting a change in at least one optical property of the indicator reagent in the presence of redox equivalents in the composition comprised to the indicator reagent. The change in the at least one optical property of the indicator reagent can than be measured. Depending on the diagnostic test element, the measurement of the change of the optical property can be achieved by different techniques described elsewhere herein in more detail. For detecting the change of an optical property such as color, a spatially resolving optical detector may be used. A spatially resolving optical detector is to be understood to mean an optical detector which has a multiplicity of optical sensors which are able to record regions of the detection side of the detection layer which are not completely congruent. More particularly, the spatially resolving optical detector can comprise at least one image sensor, i.e., an array of optical detectors which can be one-dimensional or else two-dimensional. More particularly, the optical detector can thus comprise a CCD chip and/or CMOS chip. In addition, the spatially resolving optical detector can comprise at least one optical element for imaging the detection side and/or the detection layer onto an image-sensitive surface of the spatially resolving optical detector.

A change in at least one optical property measured by the method described above shall be indicative for the presence of the analyte. It will be understood by the skilled artisan that in order to determine the amount of an analyte, it might be necessary to compare the extent of the change of the optical property. To this end, it might be, furthermore, necessary to compare a detected signal accompanying the optical change to signals accompanying optical changes elicited by known amounts of analytes, i.e. calibration signals. How such a calibration can be established is well known to the skilled artisan.

The term "amount" as used herein refers to the absolute or relative amount of analyte present in a sample applied to the diagnostic test element. A relative amount preferred according to the present invention is the concentration, i.e. the amount in relation to the volume.

### FIGURES

Fig. 1: Average values for activity are indicated compared to the amount of stabilizer (compatible solute). The activity determined in test elements without stabilizer has been subtracted.
**Fig**. **2****:** Graphic view of the stabilizing effect of ectoine and hydroxyectoine on glucose dehydrogenase and diaphorase.
Fig. 3: Graphic view of storage temperature compared to the measured glucose level as an indicator for the enzymatic activity. A) without stabilizer; B) ectoine, 1g/100g composition; C) hydroxyectoine, 1g/100g composition.
Fig. 4: Influence of different buffers used in the coating compositions. A) PO4-buffer pH 6,8, without stabilizer; after 45 days; 45°C to 4°C; B) PO4-buffer pH 6,8, 2g ectoine per 100g RF; after 45 days; 45°C to 4°C; C) HEPES pH 7,1, without stabilizer; after 45 days; 45°C to 4°C; D) HEPES pH 7,1, 2g ectoine per 100g RF; after 45 days; 45°C to 4°C
Fig. 5: Stabilizing effect of ectoine on Gluc-DOR and Gluc-DOR 31 mutant is already observable after 3 weeks at 45°C.

### EXAMPLES

### Example 1: Generation of test stripes

Four different reaction films for determining glucose levels were generated and coated on a foil essentially as described in EP 0 821 234 Example 1. The formulation of the composition is shown in the following table 1.

**Table 1: Components per 100 g of the first coating film prior to drying**

| | | | | |
|---|---|---|---|---|
| Glucosedehydrogenase from Bacillus subtilis | 1,09 g | 1,09 g | 1,09 g | 1,09 g |
| Diaphorase from Bacillus subtilis | 0,77 g | 0,77 g | 0,77 g | 0,77 g |
| NAD | 0,58 g | 0,58 g | 0,58 g | 0,58 g |
| Na/KPhosphate buffer or HEPES | 0,35g | 0,35g | 0,35g | 0,35g |
| ectoine or hydroxyectoine | 0,00 g | 1,00 q | 2,00 g | 4,00 g |
| Xanthan gum | 0.29 g | 0,29 g | 0,29 g | 0,29 g |
| silica FK 320DS | 5,80 g | 5,80 g | 5,80 g | 5,80 g |
| sodium-N-methyl-N-oleoyl-taurate | 0,03 g | 0,03 g | 0,03 g | 0,03 g |
| N-Octanoyl-N-methyl-glucamide | 0,17 g | 0,17 g | 0.17 g | 0,17 g |
| Polyvinylpyrrolidone | 0,86 g | 0,86 g | 0,86 g | 0,86 g |
| Tetraethylammoniumchloride | 0,07 g | 0,07 g | 0.07 g | 0,07 g |
| 2,18-Phosphormolybdenic acid hexasodium salt | 0,33 g | 0,33 g | 0,33 g | 0,33 g |
| Polyvinylpropionate-Dispersion (50 Gew.-% in water) | 5,00 g | 5,00 g | 5,00 g | 5,00 g |
| K3[Fe(CN)6] | 0,01 q | 0,01 g | 0,01 g | 0,01 g |
| 2-Methyl-2 butanol | 1,00 g | 1,00 g | 1,00 g | 1.00 g |
| add water up to 100g | | | | |

A pH of 6.8 was adjusted and the composition was coated as a film (about 120 micrometer) on a polycarbonate foil (125 micrometer). The coated composition was subsequently dried at 50°C.

A second coat was applied to the first coat on the foil as follows:

**Table 2: Components of the second coating film**

| | |
|---|---|
| Gantrez | 1,47 g |
| sodium-N-methyl-N-oleoyl-taurat | 0,03 g |
| PVP K25 | 2,01 g |
| Mega 8 | 0,37 g |
| Tetraethylammoniumchloride | 0,45 g |
| silica FK 320DS | 2,00 g |
| Titandioxide E171 | 22,00 g |
| Polyvinylpropionate-Dispersion (50 Gew.-% in water) | 6,25 g |
| Bis-(2-hydroxyethyl)-(4-hydroximinocyclohexa-2,5-dienylidin)-ammoniumchloride | 0,48 g |
| 2,18-Phosphormolybdenic acid hexasodium salt | 1,41 g |
| K3[Fe(CN)6] | 0,01 g |
| 2-Methyl-2 butanol | 1,00 g |
| add water up to 100g | |

A pH of 6.8 was adjusted and the composition was coated as a second film (about 25 micrometer) onto the first film coated on the foil. The coated composition was subsequently dried at 50°C. Test strips for glucose determination were generated as described in EP 0 821 234, sections ([0063 - 0067]).

### Example 2: Determination of the enzymatic activity in the test element

Test elements were stored in plastic vials in the presence of a drying agent for 6 weeks at 45°C. In a subsequent step, the test field of a test element was eluted by ultra-sonication using elution buffer. In the supernatant, enzymatic activity was determined.

**Table 3: Elution buffer and detection technique for the different enzymatic activities**

| | Elution buffer | detection technique |
|---|---|---|
| Glucosedehydrogenase | Tris/HCl, NaCl, NAD; pH 8,5 | UV-detection at 340 nm |
| Diaphorase | Tris/HCl, NaCl. Triton; pH 8,8 | INT → Tetrazolium alsz; detected at 492 nm |

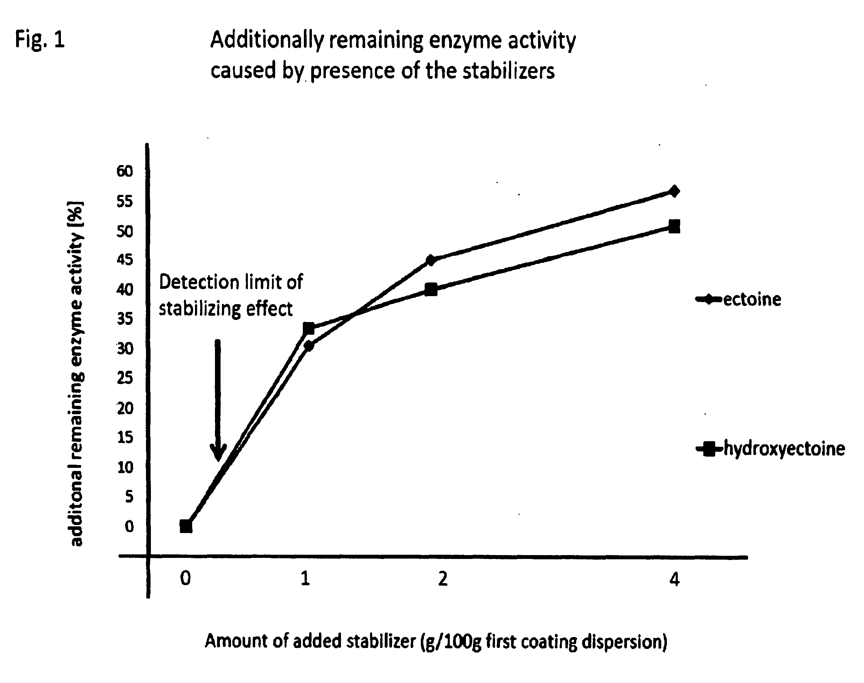

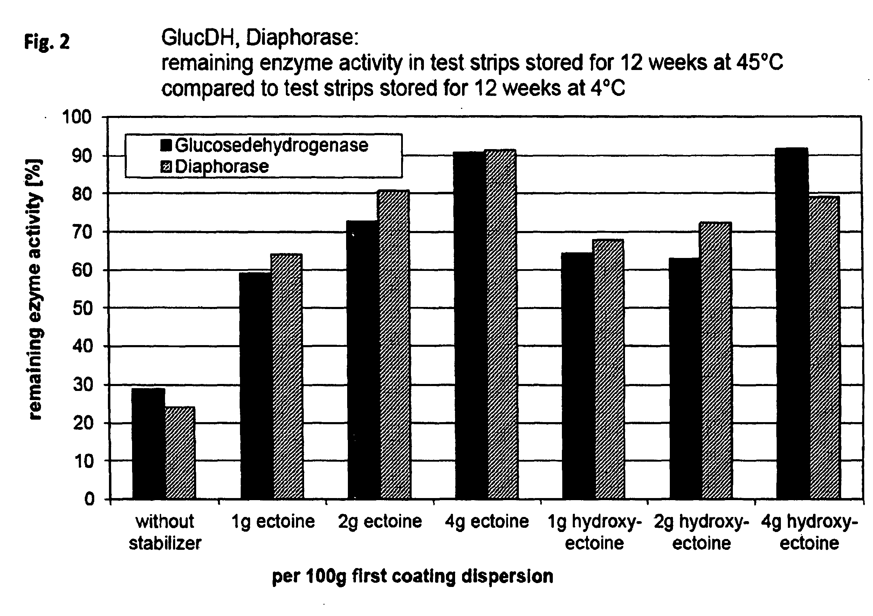

Results are shown in Fig. 1. The Figure shows that there is a significant stabilizing effect (more than 10%) for concentrations of ectoine of larger than 0.3 g per 100g coating composition. The same effect can be observed for hydroxyectoine as shown in Fig. 2. Moreover, the Figure shows that the dehydrogenase as well as the diaphorase are stabilized.

### Example 3: Determination of the enzymatic activity in the test element

Test elements were stored in plastic vials in the presence of a drying agent for 63 days at 4°C (KS), 24°C (RT), 35°C (DT), and 45°C (HT). Test elements were used to determine blood glucose levels in a plurality of venous blood samples. The samples were measured with a reference method (Hitachi) in parallel. Results were normalized with respect to the KS stored test elements.

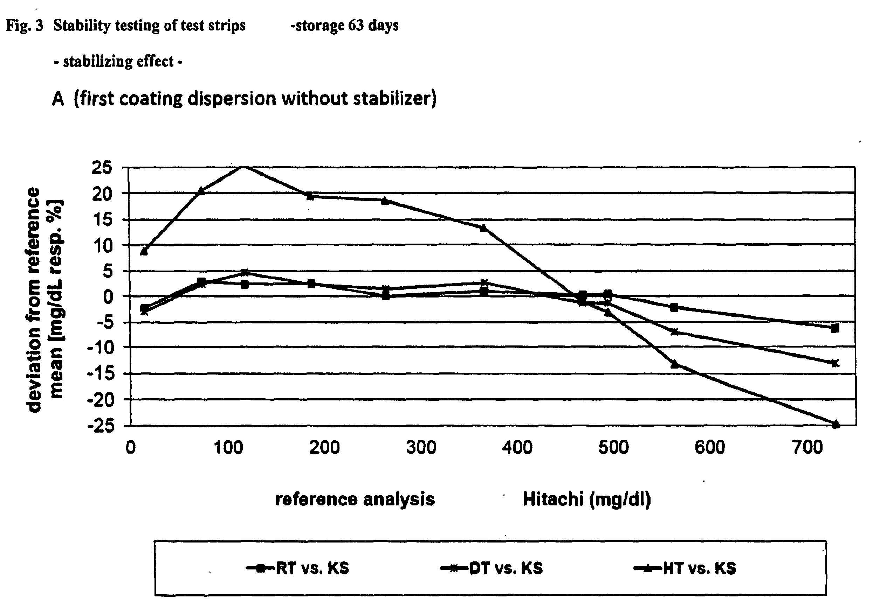

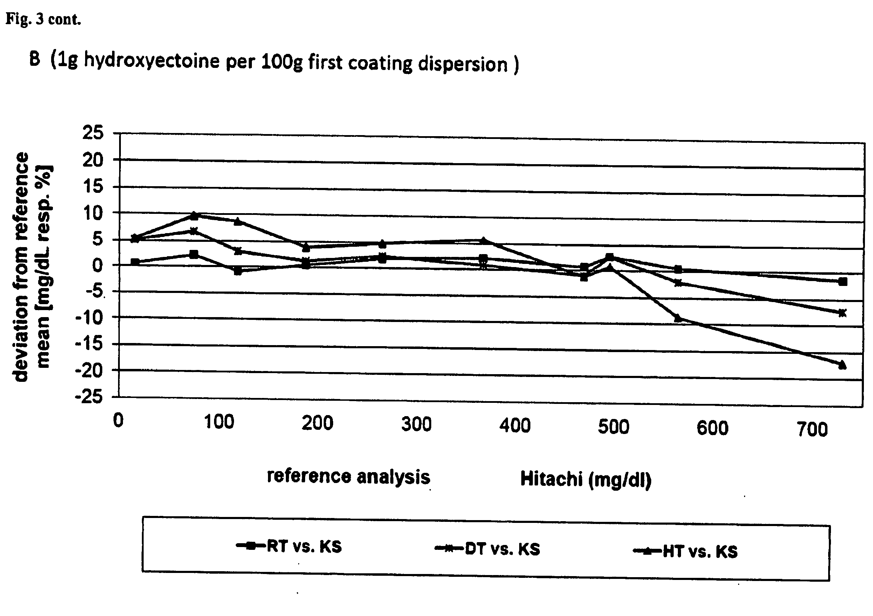

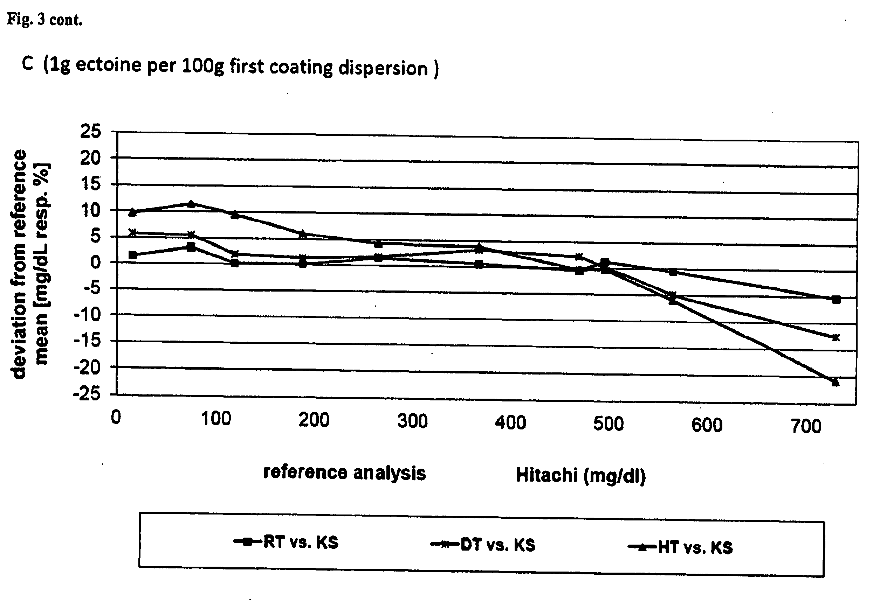

In Figure 3, the storage temperature is indicated as well as the measured glucose level as an indicator for the enzymatic activity. As is evident, ectoine and hydroxyectoine act as stabilizers and preserve the enzymatic activities.

### Example 4: Determination of the enzymatic activity in the test element with different buffers

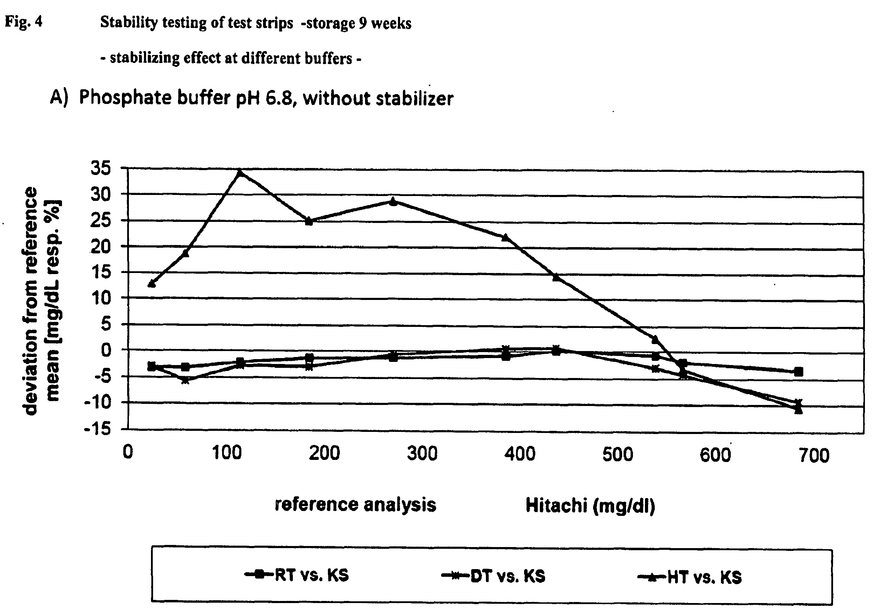

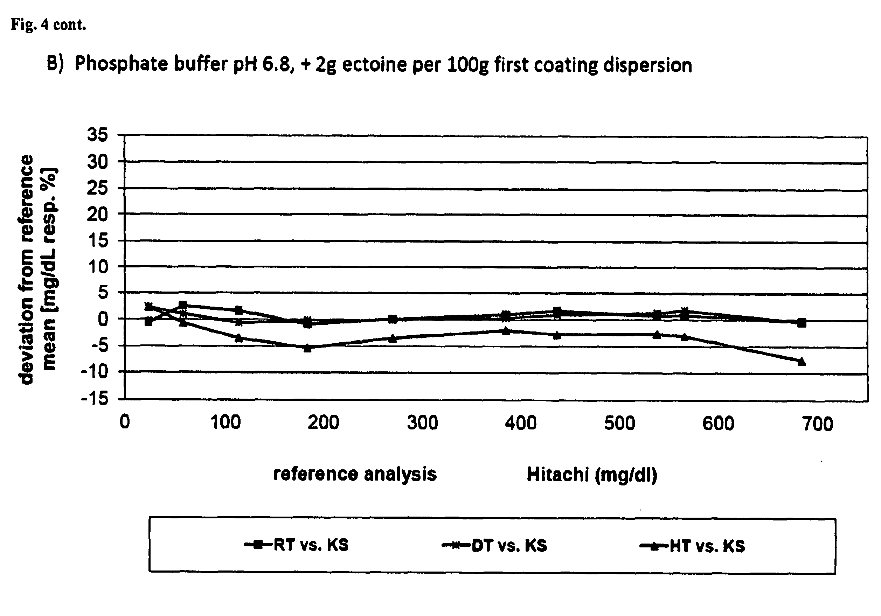

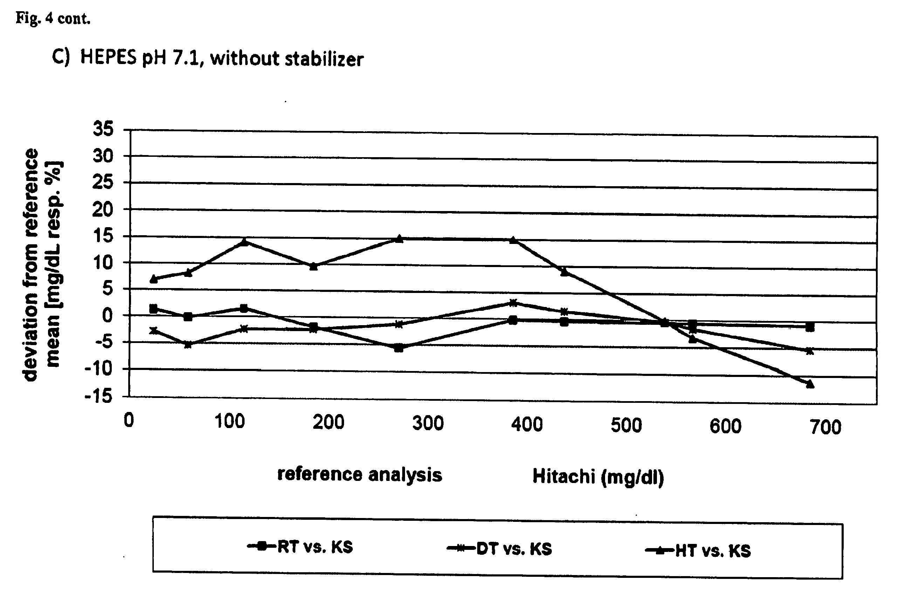

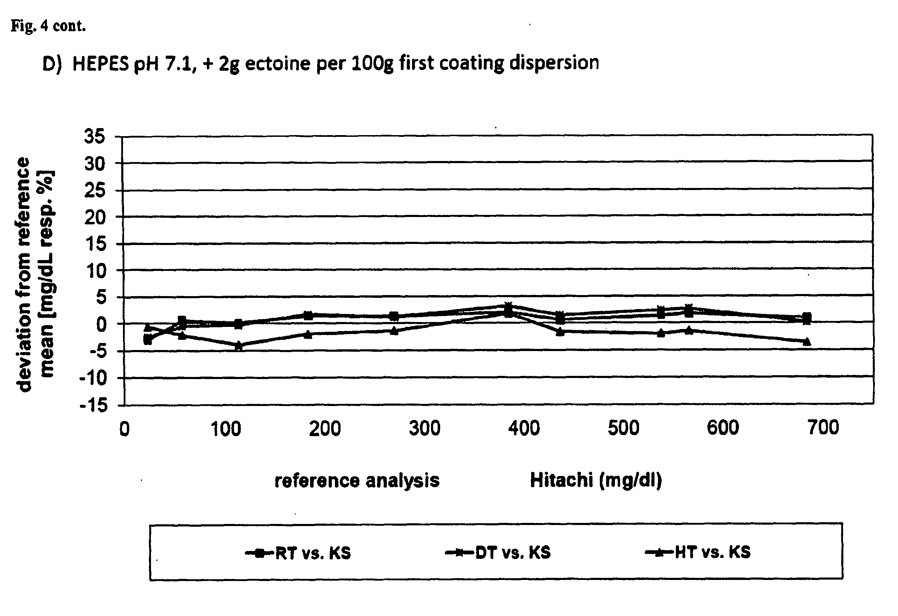

Test elements were stored and treated essentially as describe in Example 3. Different buffers, namely a phosphate-buffer pH 6,8, without stabilizer, a phosphate-buffer pH 6,8, 2g ectoine per 100g first coating film, a HEPES buffer pH 7,1, without stabilizer, and a HEPES buffer pH 7,1, 2g ectoine per 100g first coating film, were used in the coating compositions as indicated in the Figure 4. As is evident from the Figure, the stabilizing activity of ectoine is observed independent of the buffer system.

### Example 5: Determination of enzymatic activity of a glucose dehydrogenase mutant 2 in solution and dependency on ectoine or hydroxyectoine

Aliquots of a solution comprising glucose dehydrogenase mutant 2 as disclosed in WO2011/020856 were combined with different amounts of ectoine or hydroxyectoine as indicated in Table 4.

**Table 4: Stabilizing solutions**

| **Exp. No.** | | **content** | **Stabilizer and concentration** |
|---|---|---|---|
| 1 | | Glucosedehydrogenase Mut.2 3000 U/ml, NAD 10 mg/ml; K-Na Phosphate 15 mM, pH 6.8. | 0 (reference) |
| 2 | | dto | ectoine 4% (w/v) |
| 3 | | dto | ectoine 2% (w/v) |
| 4 | | dto | ectoine 1% (w/v) |
| 5 | | dto | hydroxyectoine 4% (w/v) |
| 6 | | dto | hydroxyectoine 2% (w/v) |
| 7 | | dto | hydroxyectoine 1% (w/v) |

The aliquots were stored under different storage conditions (8 days, 4°C; 8 days 35°C, 4 days 4°C followed by 4 days 45°C) and the enzymatic activity was determined after the storage. The results are shown in Table 5. No stabilizing effect of either ectoine or hydroxyectoine could be determined in solution.

**Table 5: Results**

| Exp No. | Note | | Units | 8 days at4°C | 8 days at 35°C | 4 days at 4°C, 4 days at 45°C |
|---|---|---|---|---|---|---|
| 1 | reference | | kU/g | 213 | 126 | 50 |
| 2 | ectoine appr. 4% | | kU/g | 207 | 137 | 57 |
| 3 | ectoine appr. 2% | | kU/g | 222 | 149 | 59 |
| 4 | ectoine appr. 1% | | kU/g | 222 | 144 | 60 |
| 5 | hydroxyectoine appr. 4% | | kU/g | 207 | 131 | 56 |
| 6 | hydroxyectoine appr. 2% | | kU/g | 221 | 141 | 60 |
| 7 | hydroxyectoine appr. 1% | | kU/g | 208 | 122 | 52 |

### Example 6: Assessing test elements with Gluc-DOR (=PQQ-dependent Glucose dehydrogenase)

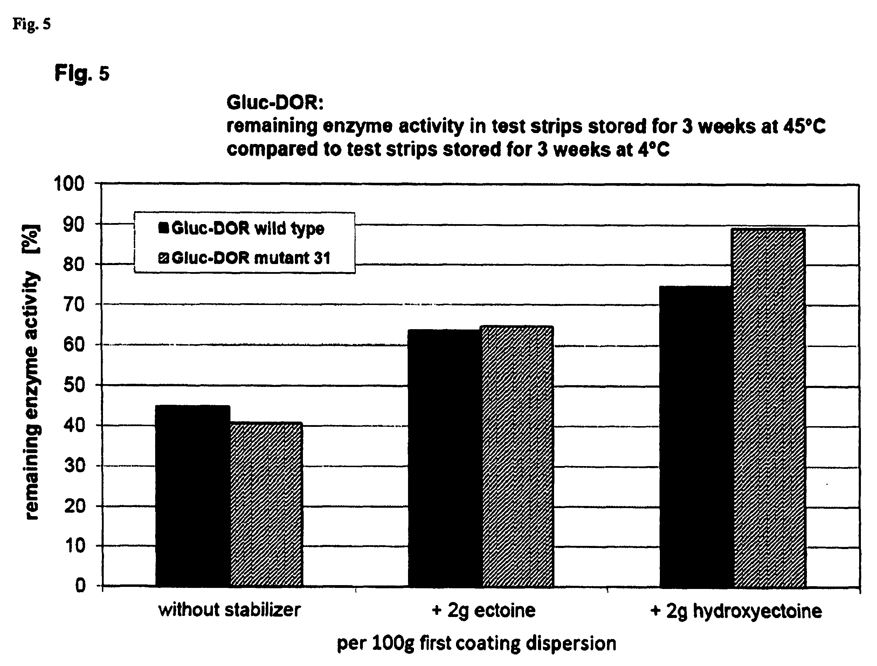

A test element with Gluc-DOR and a mutant thererof (Gluc-DOR 31) was generated as described in Example 1, above. The test element was analyzed as described in Example 2, above. The determination of the enzymatic activity was carried out using nitroso aniline as described in EP 0 620 283 B1. As is evident from Figure 5, a stabilizing effect is already observable after 3 weeks at 45°C.

## Claims

1. A dry composition comprising the components:
(a) a dehydrogenase;
(b) a redox cofactor;
(c) an agent capable of eliciting a change in at least one optical property of an indicator reagent in the presence of redox equivalents;
(d) an indicator reagent; and
(e) at least one compatible solute being ectoine or a derivative thereof,
wherein said dehydrogenase is a glucose dehydrogenase, and
wherein said derivative of ectoine is selected from the group consisting of: hydroxyectoine, homoectoine, a hydroxyectoine ester, a hydroxyectoine ether, a sulfonyl derivative of ectoine or an esterified sulfonyl derivative of ectoine and an amide of a sulfonyl derivative of ectoine.

2. The composition of claim 1, wherein said glucose dehydrogenase is selected from the group consisting of: glucose dehydrogenase (EC number 1.1.1.47), quinoprotein glucose dehydrogenase (EC number 1.1.5.2), in particular, Pyrrolo quinoline quinone (PQQ)-dependent glucose dehydrogenase (EC number 1.1.5.2), glucose-6-pospate dehydrogenase (EC number 1.1.1.49), nicotinamide adenine dinucleotide (NAD)-dependent glucose dehydrogenase (EC number 1.1.1.119) and flavin adenine dinucleotide (FAD)-dependent glucose dehydrogenase (EC number 1.1.99.10) or enzymatically active mutants thereof.

3. The composition of any one of claims 1 to 2, wherein said an agent capable of eliciting a change in the at least one optical property in the presence of redox equivalents is capable of transferring said redox equivalents from the redox cofactor to the indicator reagent.

4. The composition of claim 3, wherein said agent is (i) a diaphorase, preferably, a lipoamide deydrogenase or a NADH dehydrogenase, (ii) a phenazine, preferably, phenazinethosulfate, phenazinmethosulfate, 1-(3-carboxypropoxy)-5-ethylphenaziniumtrifluoromethansulfonate or 1-methoxyphenazinmethosulfate, (iii) a nitrosoaniline, preferably, [(4-nitrosophenyl)imino]dimethanol-hydrochloride, or (iv) a chinone, preferably, phenanthrenchinone, phenanthrolinchinone or benzo[h]-chinolinchinone.

5. The composition of any one of claims 1 to 4, wherein said redox cofactor is selected from the group consisting of: carba-NAD, NAD, FAD, and PQQ.

6. A diagnostic test element for the determination of an analyte from a body fluid sample comprising the composition of any one of claims 1 to 5 on a carrier.

7. The test element of claim 6, wherein said carrier comprises a test field containing said composition, wherein the test field has a sample application side onto which the body fluid sample is applied and a detection side which allows for detection of the change in at least one optical property of the reagent when the analyte reacts with the composition.

8. A method for the manufacture of a diagnostic test element comprising the step of generating a composition according to any one of claims 1 to 5 on a carrier.

9. The method of claim 8, wherein said generating comprises the steps of:
(i) applying a composition comprising the components (a) to (e) of the composition of the invention and a solvent (i.e. a composition comprising the components in a dissolved state rather than being dry) to a test field on the carrier; and
(ii) removing the said solvent from the composition;
or
(i) applying a composition comprising components (a), (b), (d) and (e) of the composition of the invention and a solvent (i.e. a composition comprising the components in a dissolved state rather than being dry) to a test field on the carrier in a first layer;
(ii) removing the said solvent from the composition of the first layer;
(iii) applying a composition comprising components (c) to (e) of the composition of the invention and a solvent (i.e. a composition comprising the components in a dissolved state rather than being dry) in a second layer on the first layer; and
(iv) removing the said solvent from the composition of the second layer;
or
(i) applying a composition comprising components (a), (b), (d) and (e) of the composition of the invention and a solvent (i.e. a composition comprising the components in a dissolved state rather than being dry) to a test field on the carrier in a first layer;
(ii) removing the said solvent from the composition of the first layer;
(iii) applying a composition comprising components (b) to (e) of the composition of the invention and a solvent (i.e. a composition comprising the components in a dissolved state rather than being dry) in a second layer on the first layer; and
(iv) removing the said solvent from the composition of the second layer;
or
(i) applying a composition comprising components (a), (d) and (e) of the composition of the invention and a solvent (i.e. a composition comprising the components in a dissolved state rather than being dry) to a test field on the carrier in a first layer;
(ii) removing the said solvent from the composition of the first layer;
(iii) applying a composition comprising components (b) to (e) of the composition of the invention and a solvent (i.e. a composition comprising the components in a dissolved state rather than being dry) in a second layer on the first layer; and
(iv) removing the said solvent from the composition of the second layer;
or
(i) applying a composition comprising components (c) to (e) of the composition of the invention and a solvent (i.e. a composition comprising the components in a dissolved state rather than being dry) to a test field on the carrier in a first layer;
(ii) removing the said solvent from the composition of the first layer;
(iii) applying a composition comprising components (a), (b), (d) and (e) of the composition of the invention and a solvent (i.e. a composition comprising the components in a dissolved state rather than being dry) in a second layer on the first layer; and
(iv) removing the said solvent from the composition of the second layer.
or
(i) applying a composition comprising components (b) to (e) of the composition of the invention and a solvent (i.e. a composition comprising the components in a dissolved state rather than being dry) to a test field on the carrier in a first layer;
(ii) removing the said solvent from the composition of the first layer;
(iii) applying a composition comprising components (a), (b), (d) and (e) of the composition of the invention and a solvent (i.e. a composition comprising the components in a dissolved state rather than being dry) in a second layer on the first layer; and
(iv) removing the said solvent from the composition of the second layer.
or
(i) applying a composition comprising components (b) to (e) of the composition of the invention and a solvent (i.e. a composition comprising the components in a dissolved state rather than being dry) to a test field on the carrier in a first layer;
(ii) removing the said solvent from the composition of the first layer;
(iii) applying a composition comprising components (a), (d) and (e) of the composition of the invention and a solvent (i.e. a composition comprising the components in a dissolved state rather than being dry) in a second layer on the first layer; and
(iv) removing the said solvent from the composition of the second layer.

10. The method of claim 8 or 9, wherein the compatible solute reduces a decrease of the enzymatic activity of at least one enzyme in the composition under dry conditions.

11. Use of at least one compatible solute being ectoine or a derivative thereof for reducing a decrease of the enzymatic activity of at least one enzyme in a composition under dry conditions, wherein said composition comprises a dehydrogenase, a redox cofactor, an agent capable of eliciting a change in at least one optical property of an indicator reagent in the presence of redox equivalents, and an indicator reagent,
wherein said derivative of ectoine is selected from the group consisting of:
hydroxyectoine, homoectoine, a hydroxyectoine ester, a hydroxyectoine ether, a sulfonyl derivative of ectoine or an esterified sulfonyl derivative of ectoine and an amide of a sulfonyl derivative of ectoine, and
wherein said dehydrogenase is a glucose dehydrogenase.

12. The use of claim 11, wherein said at least one compatible solute in the composition under dry conditions is comprised in a diagnostic test element, preferably, a diagnostic test element according to claim 6 or 7.

13. A method for determining the presence or amount of an analyte in a body fluid sample comprising the steps of:
(a) contacting the diagnostic test element of claim 6 or 7 with a body fluid suspected to comprise the analyte under conditions suitable for transforming the at least one enzyme to the reconstituted state;
(b) measuring a change in at least one optical property of the indicator reagent in the wetted composition comprising the at least one enzyme in the reconstituted state on the diagnostic test element, whereby the presence or amount of the analyte in the body fluid sample will be determined.

## Patentansprüche

1. Trockene Zusammensetzung, umfassend die Komponenten:
(a) eine Dehydrogenase;
(b) einen Redox-Cofaktor;
(c) ein Mittel, das fähig ist, in Gegenwart von Redoxäquivalenten eine Veränderung wenigstens einer optischen Eigenschaft eines Indikatorreagens auszulösen;
(d) ein Indikatorreagens; und
(e) wenigstens einen kompatiblen Solut, der Ectoin oder ein Derivat davon ist,
wobei die Dehydrogenase eine Glucosedehydrogenase ist und
wobei das Derivat von Ectoin ausgewählt ist aus der Gruppe bestehend aus: Hydroxyectoin, Homoectoin, einem Hydroxyectoinester, einem Hydroxyectoinether, einem Sulfonylderivat von Ectoin oder einem veresterten Sulfonylderivat von Ectoin und einem Amid eines Sulfonylderivats von Ectoin.

2. Zusammensetzung gemäß Anspruch 1, wobei die Glucosedehydrogenase ausgewählt ist aus der Gruppe bestehend aus: Glucosedehydrogenase (EC-Nummer 1.1.1.47), Quinoprotein-Glucosedehydrogenase (EC-Nummer 1.1.5.2), insbesondere Pyrrolochinolinchinon(PQQ)-abhängige Glucosedehydrogenase (EC-Nummer 1.1.5.2), Glucose-6-phosphat-Dehydrogenase (EC-Nummer 1.1.1.49), Nicotinamidadenindinucleotid (NAD) - abhängige Glucosedehydrogenase (EC-Nummer 1.1.1.119) und Flavinadenindinucleotid(FAD)-abhängige Glucosedehydrogenase (EC-Nummer 1.1.99.10) und enzymatisch aktiven Mutanten davon.

3. Zusammensetzung gemäß einem der Ansprüche 1 bis 2, wobei das Mittel, das fähig ist, in Gegenwart von Redoxäquivalenten eine Veränderung der wenigstens einen optischen Eigenschaft auszulösen, fähig ist, die Redoxäquivalente von dem Redox-Cofaktor auf das dem Indikatorreagens zu übertragen.

4. Zusammensetzung gemäß Anspruch 3, wobei das Mittel (i) eine Diaphorase, vorzugsweise eine Lipoamiddehydrogenase oder eine NADH-Dehydrogenase, (ii) ein Phenazin, vorzugsweise Phenazinethosulfat, Phenazinmethosulfat, 1-(3-Carboxypropoxy)-5-ethylphenaziniumtrifluormethansulfonat oder 1-Methoxyphenazinmethosulfat, (iii) ein Nitrosoanilin, vorzugsweise [(4-Nitrosophenyl)imino]dimethanolhydrochlorid, oder (iv) ein Chinon, vorzugsweise Phenanthrenchinon, Phenanthrolinchinon oder Benzo[h]chinolinchinon ist.

5. Zusammensetzung gemäß einem der Ansprüche 1 bis 4, wobei der Redox-Cofaktor ausgewählt ist aus der Gruppe bestehend aus: Carba-NAD, NAD, FAD und PQQ.

6. Diagnostisches Testelement für die Bestimmung eines Analyten in einer Körperflüssigkeitsprobe, umfassend die Zusammensetzung gemäß einem der Ansprüche 1 bis 5 auf einem Träger.

7. Testelement gemäß Anspruch 6, wobei der Träger ein Testfeld umfasst, das die Zusammensetzung enthält, wobei das Testfeld eine Probeauftragseite aufweist, auf die die Körperflüssigkeitsprobe aufgetragen wird, und eine Nachweisseite, die das Nachweisen der Veränderung wenigstens einer optischen Eigenschaft des Reagens erlaubt, wenn der Analyt mit der Zusammensetzung reagiert.

8. Verfahren zum Herstellen eines diagnostischen Testelements, umfassend den Schritt des Herstellens einer Zusammensetzung gemäß einem der Ansprüche 1 bis 5 auf einem Träger.

9. Verfahren gemäß Anspruch 8, wobei das Herstellen die Schritte umfasst:
(i) Aufbringen einer Zusammensetzung, die die Komponenten (a) bis (e) der Zusammensetzung gemäß der Erfindung und ein Lösungsmittel umfasst (d. h. eine Zusammensetzung, die die Komponenten in einem gelösten Zustand umfasst und nicht etwa trocken ist), auf ein Testfeld auf dem Träger; und
(ii) Entfernen des Lösungsmittels aus der Zusammensetzung;
oder
(i) Aufbringen einer Zusammensetzung, die die Komponenten (a), (b), (d) und (e) der Zusammensetzung gemäß der Erfindung und ein Lösungsmittel umfasst (d. h. eine Zusammensetzung, die die Komponenten in einem gelösten Zustand umfasst und nicht etwa trocken ist), in einer ersten Schicht auf ein Testfeld auf dem Träger;
(ii) Entfernen des Lösungsmittels aus der Zusammensetzung der ersten Schicht;
(iii) Aufbringen einer Zusammensetzung, die die Komponenten (c) bis (e) der Zusammensetzung gemäß der Erfindung und ein Lösungsmittel umfasst (d. h. eine Zusammensetzung, die die Komponenten in einem gelösten Zustand umfasst und nicht etwa trocken ist), in einer zweiten Schicht auf die erste Schicht; und
(iv) Entfernen des Lösungsmittels aus der Zusammensetzung der zweiten Schicht;
oder
(i) Aufbringen einer Zusammensetzung, die die Komponenten (a), (b), (d) und (e) der Zusammensetzung gemäß der Erfindung und ein Lösungsmittel umfasst (d. h. eine Zusammensetzung, die die Komponenten in einem gelösten Zustand umfasst und nicht etwa trocken ist), in einer ersten Schicht auf ein Testfeld auf dem Träger;
(ii) Entfernen des Lösungsmittels aus der Zusammensetzung der ersten Schicht;
(iii) Aufbringen einer Zusammensetzung, die die Komponenten (b) bis (e) der Zusammensetzung gemäß der Erfindung und ein Lösungsmittel umfasst (d. h. eine Zusammensetzung, die die Komponenten in einem gelösten Zustand umfasst und nicht etwa trocken ist), in einer zweiten Schicht auf die erste Schicht; und
(iv) Entfernen des Lösungsmittels aus der Zusammensetzung der zweiten Schicht;
oder
(i) Aufbringen einer Zusammensetzung, die die Komponenten (a), (d) und (e) der Zusammensetzung gemäß der Erfindung und ein Lösungsmittel umfasst (d. h. eine Zusammensetzung, die die Komponenten in einem gelösten Zustand umfasst und nicht etwa trocken ist), in einer ersten Schicht auf ein Testfeld auf dem Träger;
(ii) Entfernen des Lösungsmittels aus der Zusammensetzung der ersten Schicht;
(iii) Aufbringen einer Zusammensetzung, die die Komponenten (b) bis (e) der Zusammensetzung gemäß der Erfindung und ein Lösungsmittel umfasst (d. h. eine Zusammensetzung, die die Komponenten in einem gelösten Zustand umfasst und nicht etwa trocken ist), in einer zweiten Schicht auf die erste Schicht; und
(iv) Entfernen des Lösungsmittels aus der Zusammensetzung der zweiten Schicht;
oder
(i) Aufbringen einer Zusammensetzung, die die Komponenten (c) bis (e) der Zusammensetzung gemäß der Erfindung und ein Lösungsmittel umfasst (d. h. eine Zusammensetzung, die die Komponenten in einem gelösten Zustand umfasst und nicht etwa trocken ist), in einer ersten Schicht auf ein Testfeld auf dem Träger;
(ii) Entfernen des Lösungsmittels aus der Zusammensetzung der ersten Schicht;
(iii) Aufbringen einer Zusammensetzung, die die Komponenten (a), (b), (d) und (e) der Zusammensetzung gemäß der Erfindung und ein Lösungsmittel umfasst (d. h. eine Zusammensetzung, die die Komponenten in einem gelösten Zustand umfasst und nicht etwa trocken ist), in einer zweiten Schicht auf die erste Schicht; und
(iv) Entfernen des Lösungsmittels aus der Zusammensetzung der zweiten Schicht;
oder
(i) Aufbringen einer Zusammensetzung, die die Komponenten (b) bis (e) der Zusammensetzung gemäß der Erfindung und ein Lösungsmittel umfasst (d. h. eine Zusammensetzung, die die Komponenten in einem gelösten Zustand umfasst und nicht etwa trocken ist), in einer ersten Schicht auf ein Testfeld auf dem Träger;
(ii) Entfernen des Lösungsmittels aus der Zusammensetzung der ersten Schicht;
(iii) Aufbringen einer Zusammensetzung, die die Komponenten (a), (b), (d) und (e) der Zusammensetzung gemäß der Erfindung und ein Lösungsmittel umfasst (d. h. eine Zusammensetzung, die die Komponenten in einem gelösten Zustand umfasst und nicht etwa trocken ist), in einer zweiten Schicht auf die erste Schicht; und
(iv) Entfernen des Lösungsmittels aus der Zusammensetzung der zweiten Schicht;
oder
(i) Aufbringen einer Zusammensetzung, die die Komponenten (b) bis (e) der Zusammensetzung gemäß der Erfindung und ein Lösungsmittel umfasst (d. h. eine Zusammensetzung, die die Komponenten in einem gelösten Zustand umfasst und nicht etwa trocken ist), in einer ersten Schicht auf ein Testfeld auf dem Träger;
(ii) Entfernen des Lösungsmittels aus der Zusammensetzung der ersten Schicht;
(iii) Aufbringen einer Zusammensetzung, die die Komponenten (a), (d) und (e) der Zusammensetzung gemäß der Erfindung und ein Lösungsmittel umfasst (d. h. eine Zusammensetzung, die die Komponenten in einem gelösten Zustand umfasst und nicht etwa trocken ist), in einer zweiten Schicht auf die erste Schicht; und
(iv) Entfernen des Lösungsmittels aus der Zusammensetzung der zweiten Schicht.

10. Verfahren gemäß Anspruch 8 oder 9, wobei der kompatible Solut eine Abnahme der enzymatischen Aktivität von wenigstens einem Enzym in der Zusammensetzung unter trockenen Bedingungen verringert.

11. Verwendung von wenigstens einem kompatiblen Solut, der Ectoin oder ein Derivat davon ist, zum Verringern einer Abnahme der enzymatischen Aktivität von wenigstens einem Enzym in einer Zusammensetzung unter trockenen Bedingungen, wobei die Zusammensetzung eine Dehydrogenase, einen Redox-Cofaktor, ein Mittel, das fähig ist, in Gegenwart von Redoxäquivalenten eine Veränderung wenigstens einer optischen Eigenschaft eines Indikatorreagens auszulösen, und ein Indikatorreagens umfasst,
wobei das Derivat von Ectoin ausgewählt ist aus der Gruppe bestehend aus: Hydroxyectoin, Homoectoin, einem Hydroxyectoinester, einem Hydroxyectoinether, einem Sulfonylderivat von Ectoin oder einem veresterten Sulfonylderivat von Ectoin und einem Amid eines Sulfonylderivats von Ectoin, und
wobei die Dehydrogenase eine Glucosedehydrogenase ist.

12. Verwendung gemäß Anspruch 11, wobei der wenigstens eine kompatible Solut in der Zusammensetzung unter trockenen Bedingungen in einem diagnostischen Prüfelement enthalten ist, vorzugsweise einem diagnostischen Testelement gemäß Anspruch 6 oder 7.

13. Verfahren zum Bestimmen des Vorhandenseins oder der Menge eines Analyten in einer Körperflüssigkeitsprobe, umfassend die Schritte:
(a) Inkontaktbringen des diagnostischen Testelements gemäß Anspruch 6 oder 7 mit einer Körperflüssigkeit, von der vermutet wird, dass sie den Analyten umfasst, unter Bedingungen, die zum Umwandeln des wenigstens einen Enzyms in den rekonstituierten Zustand geeignet sind;
(b) Messen einer Veränderung der wenigstens einen optischen Eigenschaft des Indikatorreagens in der befeuchteten Zusammensetzung, die das wenigstens eine Enzym in dem rekonstituierten Zustand umfasst, auf dem diagnostischen Testelement, wodurch das Vorhandensein oder die Menge des Analyten in der Körperflflüssigkeitsprobe bestimmt werden wird.

## Revendications

1. Composition sèche comprenant les constituants suivants :
(a) une déshydrogénase ;
(b) un cofacteur redox ;
(c) un agent susceptible d'induire un changement dans au moins une propriété optique d'un réactif indicateur en présence d'équivalents redox ;
(d) un réactif indicateur ; et
(e) au moins un soluté compatible étant l'ectoïne ou un dérivé de celle-ci,
ladite déshydrogénase étant une glucose déshydrogénase, et
ledit dérivé d'ectoïne étant choisi dans le groupe constitué par : l'hydroxyectoïne, l'homoectoïne, un ester d'hydroxyectoïne, un éther d'hydroxyectoïne, un dérivé sulfonylé d'ectoïne ou un dérivé sulfonylé estérifié d'ectoïne et un amide d'un dérivé sulfonylé d'ectoïne.

2. Composition de la revendication 1, dans laquelle ladite glucose déshydrogénase est choisie dans le groupe constitué par : la glucose déshydrogénase (numéro EC 1.1.1.47), la quinoprotéine glucose déshydrogénase (numéro EC 1.1.5.2), en particulier la glucose déshydrogénase dépendante de la pyrroloquinoline-quinone (PQQ) (numéro EC 1.1.5.2), la glucose-6-phosphate déshydrogénase (numéro EC 1.1.1.49), la glucose déshydrogénase dépendante du nicotinamide adénine dinucléotide (NAD) (numéro EC 1.1.1.119) et la glucose déshydrogénase dépendante de la flavine adénine dinucléotide (FAD) (numéro EC 1.1.99.10) ou des mutants enzymatiquement actifs de celles-ci.

3. Composition de l'une quelconque des revendications 1 à 2, dans laquelle ledit agent susceptible d'induire un changement dans l'au moins une propriété optique en présence d'équivalents redox est susceptible de transférer lesdits équivalents redox du cofacteur redox au réactif indicateur.

4. Composition de la revendication 3, dans laquelle ledit agent est (i) une diaphorase, de préférence une lipoamide déshydrogénase ou une NAD déshydrogénase, (ii) une phénazine, de préférence l'éthosulfate de phénazine, le méthosulfate de phénazine, le trifluorométhanesulfonate de 1-(3-carboxypropoxy)-5-éthylphénazinium ou l'éthosulfate de 1-méthoxyphénazine, (iii) une nitrosoaniline, de préférence le chlorhydrate de [(4-nitrosophényl)imino]-diméthanol, ou (iv) une quinone, de préférence la phénanthrènequinone, la phénanthrolinequinone ou la benzo[h]-quinolinequinone.

5. Composition de l'une quelconque des revendications 1 à 4, dans laquelle ledit cofacteur redox est choisi dans le groupe constitué par : le carba-NAD, le NAD, la FAD, et la PQQ.

6. Élément de test de diagnostic pour la détermination d'un analyte issu d'un échantillon de fluide corporel comprenant la composition de l'une quelconque des revendications 1 à 5 sur un support.

7. Élément de test de la revendication 6, dans lequel ledit support comprend un champ de test contenant ladite composition, le champ de test ayant un côté application d'échantillon sur lequel l'échantillon de fluide corporel est appliqué et un côté détection qui permet la détection du changement dans au moins une propriété optique du réactif quand l'analyte réagit avec la composition.

8. Procédé de fabrication d'un élément de test de diagnostic comprenant l'étape de préparation d'une composition selon l'une quelconque des revendications 1 à 5 sur un support.

9. Procédé de la revendication 8, dans lequel ladite préparation comprend les étapes consistant à :
(i) appliquer une composition comprenant les constituants (a) à (e) de la composition de l'invention et un solvant (c.-à-d. une composition comprenant les constituants dans un état dissous plutôt que secs) à un champ de test sur le support ; et
(ii) retirer ledit solvant de la composition ;
ou
(i) appliquer une composition comprenant les constituants (a), (b), (d) et (e) de la composition de l'invention et un solvant (c.-à-d. une composition comprenant les constituants dans un état dissous plutôt que secs) à un champ de test sur le support en une première couche ;
(ii) retirer ledit solvant de la composition de la première couche ;
(iii) appliquer une composition comprenant les constituants (c) à (e) de la composition de l'invention et un solvant (c.-à-d. une composition comprenant les constituants dans un état dissous plutôt que secs) en une deuxième couche sur la première couche ; et
(iv) retirer ledit solvant de la composition de la deuxième couche ;
ou
(i) appliquer une composition comprenant les constituants (a), (b), (d) et (e) de la composition de l'invention et un solvant (c.-à-d. une composition comprenant les constituants dans un état dissous plutôt que secs) à un champ de test sur le support en une première couche ;
(ii) retirer ledit solvant de la composition de la première couche ;
(iii) appliquer une composition comprenant les constituants (b) à (e) de la composition de l'invention et un solvant (c.-à-d. une composition comprenant les constituants dans un état dissous plutôt que secs) en une deuxième couche sur la première couche ; et
(iv) retirer ledit solvant de la composition de la deuxième couche ;
ou
(i) appliquer une composition comprenant les constituants (a), (d) et (e) de la composition de l'invention et un solvant (c.-à-d. une composition comprenant les constituants dans un état dissous plutôt que secs) à un champ de test sur le support en une première couche ;
(ii) retirer ledit solvant de la composition de la première couche ;
(iii) appliquer une composition comprenant les constituants (b) à (e) de la composition de l'invention et un solvant (c.-à-d. une composition comprenant les constituants dans un état dissous plutôt que secs) en une deuxième couche sur la première couche ; et
(iv) retirer ledit solvant de la composition de la deuxième couche ;
ou
(i) appliquer une composition comprenant les constituants (c) à (e) de la composition de l'invention et un solvant (c.-à-d. une composition comprenant les constituants dans un état dissous plutôt que secs) à un champ de test sur le support en une première couche ;
(ii) retirer ledit solvant de la composition de la première couche ;
(iii) appliquer une composition comprenant les constituants (a), (b), (d) et (e) de la composition de l'invention et un solvant (c.-à-d. une composition comprenant les constituants dans un état dissous plutôt que secs) en une deuxième couche sur la première couche ; et
(iv) retirer ledit solvant de la composition de la deuxième couche ;
ou
(i) appliquer une composition comprenant les constituants (b) à (e) de la composition de l'invention et un solvant (c.-à-d. une composition comprenant les constituants dans un état dissous plutôt que secs) à un champ de test sur le support en une première couche ;
(ii) retirer ledit solvant de la composition de la première couche ;
(iii) appliquer une composition comprenant les constituants (a), (b), (d) et (e) de la composition de l'invention et un solvant (c.-à-d. une composition comprenant les constituants dans un état dissous plutôt que secs) en une deuxième couche sur la première couche ; et
(iv) retirer ledit solvant de la composition de la deuxième couche ;
ou
(i) appliquer une composition comprenant les constituants (b) à (e) de la composition de l'invention et un solvant (c.-à-d. une composition comprenant les constituants dans un état dissous plutôt que secs) à un champ de test sur le support en une première couche ;
(ii) retirer ledit solvant de la composition de la première couche ;
(iii) appliquer une composition comprenant les constituants (a), (d) et (e) de la composition de l'invention et un solvant (c.-à-d. une composition comprenant les constituants dans un état dissous plutôt que secs) en une deuxième couche sur la première couche ; et
(iv) retirer ledit solvant de la composition de la deuxième couche.

10. Procédé de la revendication 8 ou 9, dans lequel le soluté compatible réduit une diminution de l'activité enzymatique d'au moins une enzyme dans la composition dans des conditions sèches.

11. Utilisation d'au moins un soluté compatible étant l'ectoïne ou un dérivé de celle-ci pour la réduction d'une diminution de l'activité enzymatique d'au moins une enzyme dans une composition dans des conditions sèches, ladite composition comprenant une déshydrogénase, un cofacteur redox, un agent susceptible d'induire un changement dans au moins une propriété optique d'un réactif indicateur en présence d'équivalents redox, et un réactif indicateur,
ledit dérivé d'ectoïne étant choisi dans le groupe constitué par : l'hydroxyectoïne, l'homoectoïne, un ester d'hydroxyectoïne, un éther d'hydroxyectoïne, un dérivé sulfonylé d'ectoïne ou un dérivé sulfonylé estérifié d'ectoïne et un amide d'un dérivé sulfonylé d'ectoïne, et
ladite déshydrogénase étant une glucose déshydrogénase.

12. Utilisation de la revendication 11, dans laquelle ledit au moins un soluté compatible dans la composition dans des conditions sèches est contenu dans un élément de test de diagnostic, de préférence un élément de test de diagnostic selon la revendication 6 ou 7.

13. Procédé de détermination de la présence ou quantité d'un analyte dans un échantillon de fluide corporel comprenant les étapes consistant à :
(a) mettre l'élément de test de diagnostic de la revendication 6 ou 7 en contact avec un fluide corporel suspecté de comprendre l'analyte dans des conditions appropriées pour transformer l'au moins une enzyme vers l'état reconstitué ;
(b) mesurer un changement dans au moins une propriété optique du réactif indicateur dans la composition mouillée comprenant l'au moins une enzyme dans l'état reconstitué sur l'élément de test de diagnostic, par lequel la présence ou quantité de l'analyte dans l'échantillon de fluide corporel sera déterminée.
